# EUROPEAN PATENT SPECIFICATION

(11) **EP 0 684 786 B1**
(45) Date of publication and mention of the grant of the patent: **01.10.2003**
(21) Application number: 94909815.6
(22) Date of filing: 10.02.1994
(51) Int. Cl.: A61B 6/12

(54) **FIDUCIAL MARKER**
MARKIERUNG ALS BEZUGSPUNKT
REPERE DE CALAGE

(30) Priority: 12.02.1993 US 17167
(43) Date of publication of application: 06.12.1995
(60) Divisional application: 02077343.8
(73) Proprietor: Allen, George S., Nashville, TN 37205 (US)
(72) Inventor: McCrory, Jennifer J., Lincoln, RI 02865 (US); Fitzpatrick, John Michael, Nashville, TN 37205 (US); Willcott, M. Robert, Houston, TX 77602-2338 (US); Maciunas, Robert J., Nashville, TN 37214 (US); Maurer, Calvin R. Jr., Nashville, TN 37212 (US); Allen, George S., Nashville, TN 37205 (US)
(74) Representative: Ede, Eric
(86) International application number: PCT/US94/02083
(87) International publication number: WO 94/017733

(56) References cited:
- EP-A- 0 146 699
- EP-A- 0 427 358
- EP-A- 0 591 712
- US-A- 4 618 978
- US-A- 4 991 579

## Description

### Background of the Invention

Recent years have seen the development of diagnostic techniques that allow the practicing clinician to obtain high fidelity views of the anatomical structure of the human body. Imaging systems such as computed tomographic (CT) x-ray imagers, positron emission tomographic (PET) scanners, single photon emission computed tomography (SPECT) scanners and nuclear magnetic resonance imaging (MRI) machines have provided clinicians with the ability to improve visualization of the anatomical structure of the human body without surgery or other invasive techniques. In lieu of exploratory surgery, the patient can be subjected to the scanning modalities of such imaging systems, and the patient's anatomical structure can be reproduced in a form for evaluation by a trained doctor.

The doctor sufficiently experienced in these techniques can evaluate the images of the patient's anatomy and determine if there are any abnormalities present. An abnormality in the form of a lesion appears on the image as a shape that has a discernable contrast with the surrounding area. The difference in contrast is due to the lesion having imaging properties that differ from those of the surrounding body tissue. Moreover, the contrasting shape that represents the lesion appears at a location on the image where such a shape would not normally appear with regard to a similar image of a healthy person.

Once a lesion has been identified, several methods of treatment are utilized to remove or destroy the lesion, including chemotherapy, radiation therapy, and surgery. When chemotherapy is chosen, drugs are introduced into the patient's body to destroy the lesion. During the course of treatment, imagers are commonly used to follow the progress of treatment by subjecting the patient to periodic scans and comparing the images taken over the course of the treatment to ascertain any changes in the lesion configurations.

In radiation therapy, the images of the lesion generated by the imager are used by a radiologist to adjust the irradiating device and to direct radiation solely at the lesion while minimizing or eliminating adverse effects to surrounding healthy tissue. During the course of the radiation treatment, the imaging system is also used to follow the progress of the patient in the same manner described above with respect to chemotherapy.

When surgery is used to remove a lesion or other abnormality, the images of the lesion in the patient can guide the surgeon during the operation. By reviewing the images prior to surgery, the surgeon can decide the best strategy for reaching and biopsying, excising, or otherwise manipulating the abnormality or lesion, whether it is a brain tumor, arteriovenous malformation, infection or other entity. After surgery has been performed, further scanning is utilized to evaluate the success of the surgery and the subsequent progress of the patient.

A problem associated with the scanning techniques mentioned above concerns the accurate selection and comparison of views of identical areas in images that have been obtained by imagers at different times or by images obtained essentially at the same time using different image modalities, e.g., CT, MRI, SPECT, and PET. This problem has two aspects. First, in order to relate the information in an image of the anatomy to the anatomy itself, it is necessary to establish a one-to-one mapping between points in the image and points on the anatomy. This is referred to as registering image space to physical space.

The second aspect concerns the registration of one image space to another image space. The goal of registering two arbitrarily oriented three dimensional images is to align the coordinate systems of the two images such that any given point in the scanned anatomy is assigned identical addresses in both images. The calculation of the rigid body transformation necessary to register the two coordinate systems requires knowledge of the coordinate vectors of at least three points in the two systems. Such points are called "fiducial points" or "fiducials," and the fiducials used are the geometric centers of markers, which are called "fiducial markers". These fiducials are used to correlate image space to physical space and to correlate one image space to another image space. The fiducial markers provide a constant frame of reference visible in a given imaging mode to make registration possible. The general technique for using fiducial markers to obtain registration of image data across time is set forth in U.S. Patent No. 4,991,579 to George S. Allen, the contents of which are incorporated herein by reference.

One problem extant in the field lies in the provision of fiducials capable of use with several imaging modalities. MRI and X-ray CT images are digital images, in which the images are formed point by point. These points are called picture elements, or pixels, and are associated with an intensity of light emitted from a cathode ray tube, or are used to form an image on film. The array of lighted pixels enables the observer to view an image. The manner in which the intensity of any given pixel is altered or modulated varies with the imaging modality employed. In X-ray CT, such modulation is a function primarily of the number of electrons per unit volume being scanned. In MR imaging, the parameters primarily influencing this modulation are the proton spin density and longitudinal and transverse relaxation times T1 and T2, which are also known as the spin-lattice and spin-spin relaxation times, respectively. In constructing a fiducial marker, one must be aware that an agent that can be imaged under one imaging modality will not necessarily be imageable under another modality. And yet, the ability to image under both CT and MBI with a given marker would be especially useful, in that one would then be able to register images derived from different imaging modalities. For example, the capability to register CT and MR images would allow the integration of information concerning bony structure provided by a CT scan with the soft tissue anatomical information provided by an MRI scan. The closest prior art EP 0427358 describes an interactive system for guiding the use of a surgical tool using at least one imaging technique such as CT scanning. The mechanical arm has a fixed base a the first end and tool holder that holds the surgical tool at the second end. A display displays one or more images from the image space of a patients anatomy. The computer is coupled to the display and mechanical arm. The computer attracts the location of the surgical tool through physical space, performs a transforming rotation of the physical space to the image space, and causes the display to display the location of the surgical tool within the image base. However, there remains a need for a fiducial marker that can be used to establish a known coordinate system under several imaging modalities.

US4991579 describes a fudicial implant for the human body which is detectable by an imaging system. The implant comprises a first portion and a second portion, the first portion configured to be detected by an imaging system and placed beneath the skin. The second portion is configured for fixed attachment to a bone beneath the skin without penetrating entirely through the bone and without fracturing the bone. Additionally, the placement of three fudicial markers into a portion of anatomy of the human body allows for the recreation of a particular image slice of the portion of the anatomy taken by an imaging system with respect to a first time period, at subsequent imaging sessions and also with different scan modalities. This provides a doctor with the ability to accurately follow the progress of the portion of the anatomy of interest. Moreover, the existence of three fudicial implants allows a target to be identified within the portion of anatomy relative to an external co-ordinate system. The portion of anatomy which the target may then be operated on, for instance, robotically, or precisely irrigated.

A further problem in the field arises from the competing needs of accommodating patient comfort, which would tend to lead clinicians toward the minimization of marker size, with the desire of clinicians to use markers that are as bright and thus as large as possible. Such brightness is desirable because it provides a strong signal that can be distinguished from noise inherent in the imaging process. The use of large-sized markers is also desirable so that the image of the marker occupies as many pixels as possible. Increasing the number of pixels occupied by the marker increases the accuracy with which the position of the marker can be determined. Furthermore, the general technique of using fiducial markers requires the determination of the centroid of the marker; it is easier to compute the centroid for a large, bright marker than for a smaller, dimmer marker. On the other hand, the larger the marker is, the more difficult it is for the patient to tolerate its presence for extended periods of time. There remains a need for a marker which can exploit the advantages presented by increased size that would also be tolerated by the patient during the period of its use. There is also a need for a small multi-modality marker that can be implanted into a patient and remain there for more extended periods of time. Such a more permanent fiducial marker would preferably be detectable by a non-invasive technique so that its position in physical space could be determined and its centroid computed even as it remained hidden from visual inspection beneath the patient's skin.

### Summary of the Invention

In view of the foregoing needs, the present invention provides medical workers with fiducial markers that can be imaged under a variety of imaging modalities, i.e., are multi-modal. The markers can be used to register image space onto image space across imaging modalities. The fiducial marker may also be used for the registration of imaging space and physical space for the successful performance of image guided craniotomies, biopsies, cyst aspirations, radiation therapy, ventricular shunt placements, and other similar surgeries.

According to the present invention there is provided a fiducial marker assembly, the assembly comprising; an imaging marker having a non metallic housing of a biocompatible material, the housing including a cavity containing a mixture of agents, wherein the mixture comprises two agents which constitute respective imaging materials for mutually different imaging modalities, and in that the respective centroids of said respective imaging materials are substantially coincident.

According to the present invention there is also provided a fiducial marker assembly, the assembly comprising; an imaging marker having a non metallic housing of a biocompatible material, and doped with a first agent, the housing further including a cavity containing a second agent, wherein both agents each constitute respective imaging materials for mutually different imaging modalities, and in that the respective centroids of said respective imaging materials are substantially coincident.

Preferred embodiments of the present invention are further defined in the claims hereafter.

The fiducial marker includes a hollow container, preferably cylindrical or spherical, that may be made of imageable material and which is filled with liquids suitable for various imaging modalities. In one version, the marker is of suitably compact size and shape to be implanted into bone for periods of prolonged duration that may be measured in years. A cylindrical shape is preferred, as it minimizes the size of the incision required for insertion by maximizing the available volume of contrast agent for a given incision size. A permanently implanted marker allows comparison of scans over time for follow-up therapy (for example, to make lesion volume comparisons in order to monitor growth). It also allows fractionated radiotherapy, in which small doses of radiation are administered frequently over the course of treatment.

One known measure for defining known set of points about the human skull for this purpose involves the use of a stereotactic frame (see U.S. Patent No. 4,608,977 for a general description of such a device). At present, a stereotactic frame cannot be used for this therapy because the frame poses a significant risk of infection, is too painful, or is too bulky and restrictive to be left on for an extended time and cannot be re-attached in the same location to tolerance of submillimetric accuracy. This problem is resolved by the use of an implantable marker that can be well tolerated by the patient for extended periods of time. Such an implantable fiducial marker can also be localized in the radiation therapy suite and thereby enable the patient's image space, intracranial physical space, and radiation therapy device to be registered with each other.

Alternatively, the fiducial marker may take the form of a relatively larger temporary marker that is removably attached to a base that is rigidly affixed to bone. In this case, the base portion is left in place for a period of days or weeks and is provided with means for detachably receiving an imaging marker. By permitting the removal of the imaging marker after scanning, the over-all height profile of the subcutaneous marker base is reduced, adding to its overall stability during implantation. In this way, the imaging marker, which need only be kept in place attached to its base for the few hours required for the medical procedure, can be made larger than could otherwise be tolerated in the case of markers left in place for days on end. The larger imaging marker produces a brighter image and is easier to localize in image space than would be the case for a smaller marker.

The interchangeable nature of this marker also makes it suitable for use with PET and/or SPECT scans. In both of these modalities, an image marker must be radioactive. Furthermore, in both of these modalities, it is necessary to obtain with each imaging scan a so-called "transmission scan," in which no radioactive substance is present. The transmission scan must be obtained with the patient in the same position as the imaging scan, and it is therefore not feasible for the marker bases to be implanted between scans. Instead, it is necessary to attach a nonradioactive marker for the transmission scan and then to replace the nonradioactive marker with a radioactive one for the imaging scan. The visibility of the markers in CT, MRI, PET, and SPECT images allows one to register images obtained with any of these modalities.

In both the temporary and the more permanent versions of the marker, the container is charged with aqueous imaging agents to provide imaging capability in MRI. CT imaging capability may be provided in either of two ways: by doping the plastic housing with agents that will render the marker housing imageable under CT, in which case the shape of the housing so doped is such that its volume centroid is coincident with the center of the volume occupied by the MRI imaging agent, or by mixing the aqueous MRI imaging agents with other aqueous agents imageable under CT. Additionally, both ways may be employed in the same marker. The imaging agents are selected so as to provide suitable imaging in both modalities and, where an aqueous CT imaging agent is employed, must be miscible. The use of a miscible liquid combination results in the same volume being visible in different imaging modalities with coincident centers for the purpose of locating the center of the marker. In the case of PET and/or SPECT, an external marker is filled with the appropriate radioisotope and used in place of the MRI/CT markers described in the previous paragraph. In an alternative employing the temporary fiducial marker, a kit of markers can be provided in which each marker is optimized for one imaging modality (MRI, CT, PET, or SPECT).

### Brief Description of the Drawings

For a more complete understanding of this invention, reference should now be made to the embodiments illustrated in greater detail in the accompanying drawings and described below. In the drawings:
FIG. 1A is an exploded perspective view of a temporary fiducial marker embodying features of the present invention, in which the imaging marker is shown separated from the base with respect to which it can releasably be attached;
FIG. 1B shows the marker of FIG. 1a as assembled;
FIG. 2A is an elevational view of the fiducial marker assembly, in which the imaging marker is shown attached to the base;
FIG. 2B illustrates in cross section the invention shown in FIG. 2A as viewed along line A-A;
FIG. 3A is an elevational view of the base portion of the invention;
FIG. 3B is a view of the top portion of the base ;
FIG. 3C is a cross sectional view of the base taken along line C-C showing the grooves that receive the imaging marker;
FIG. 4A is an elevational view of the cap portion of the imaging marker;
FIG. 4B is a top plan view of the cap portion shown in FIG. 4A;
FIG. 4C is a bottom plan view of the cap; and
FIG. 5 is a cross sectional view of a different version of the invention.

### Detailed Description

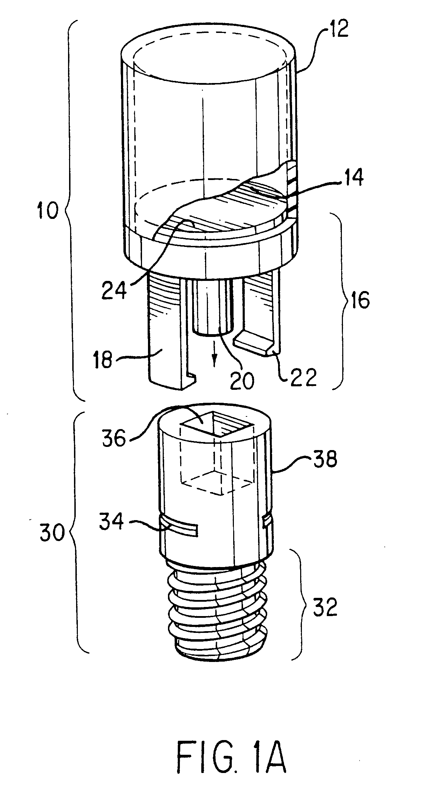

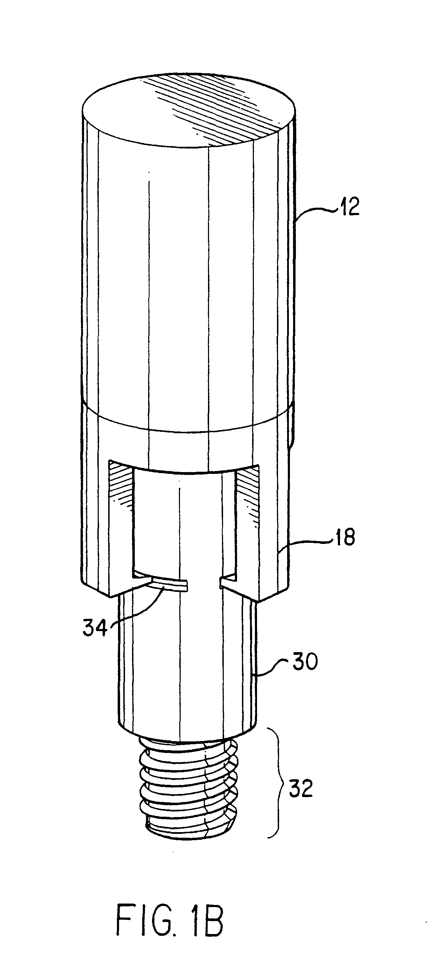

Referring now specifically to the drawings, wherein like numerals indicate like parts throughout, a version of a temporary fiducial marker assembly is indicated in FIGS. 1 - 4. These figures illustrate a fiducial marker assembly comprising an imaging marker 10 and a base 30.

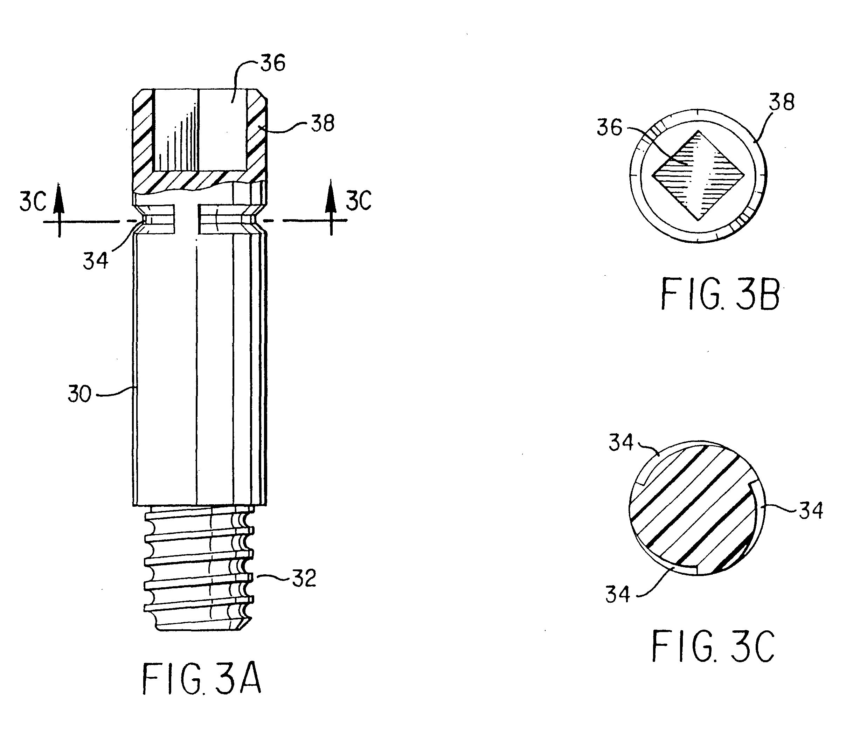

The base 30 has a threaded portion 32 at a first end. The threads enable a surgeon to securely attach the base into the skull or other desired portion of bone tissue. Other connecting structure is provided to securely and releasably link the imaging marker with the base. For example, in the illustrated embodiment, the end of the base opposite the threaded portion terminates in a socket head 38 which contains a socket-like recess 36. (It is anticipated that the base will be implanted into bone with the aid of an insertion tool that twists the base into the bone or into a hole provided in the bone. The recess is non-circular so as to better transmit the torque provided by such an insertion tool.) Just beneath the socket head 38 are a plurality (as seen in FIG. 3C, three) of grooves 34. As shall be further explained below, the socket 38 and the grooves 34 provide for the secure and releasable attachment of the imaging marker portion with base portion.

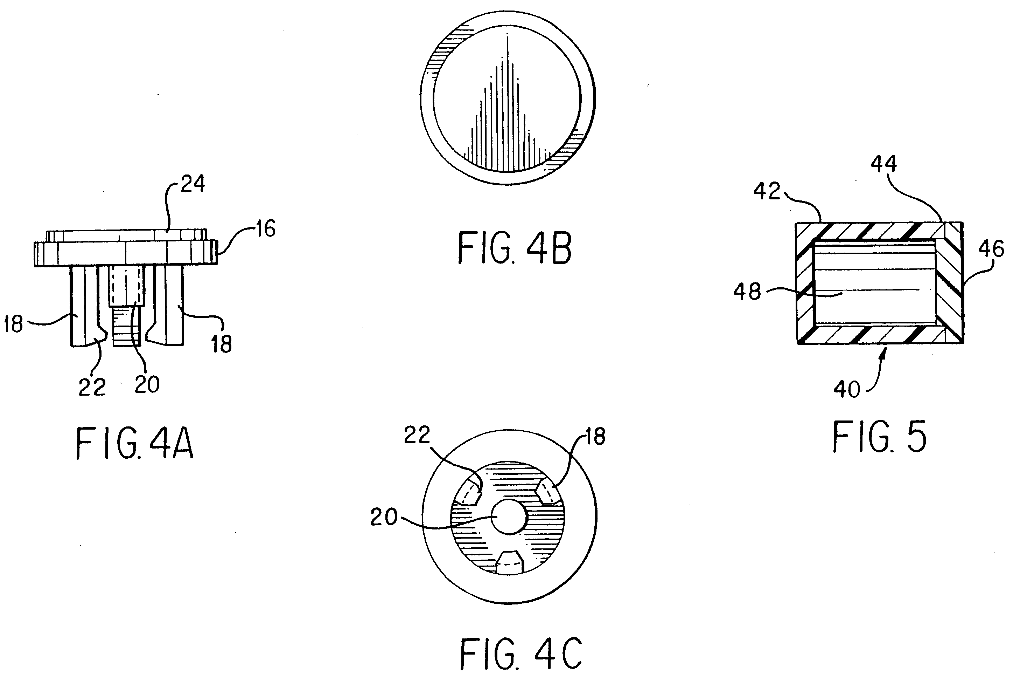

The imaging marker portion of the temporary fiducial marker assembly may consist of two principal portions, a cylinder 12 and a cap 16 (see FIGS. 4A - 4C). The cylinder 12 contains a cavity 14 for receiving a mixture of imaging agents whose composition is determined by the imaging modalities to be employed. While in this version, the vessel containing the imaging agents is preferably cylindrical so as to simplify the process by which the centroid of the corresponding volume of imaging agent is determined, other shapes (such as a box or sphere) could be employed as well. The cylinder 12 is closed at one end and open at the other to allow for the introduction of the imaging agents. In one version of the device, a cap 16 is used to seal off the open end of the cylinder once the imaging agents have been added to the cylinder. In this version, the cap may be cemented or welded into place. The cap may be provided with a plug portion 24 that protrudes into and thereby helps seal off the cavity 14 of the cylinder 12 against leakage of the imaging agents. Other conventional filling and sealing techniques, such as blow-molding, ultrasonic welding, or heat sealing, may be used.

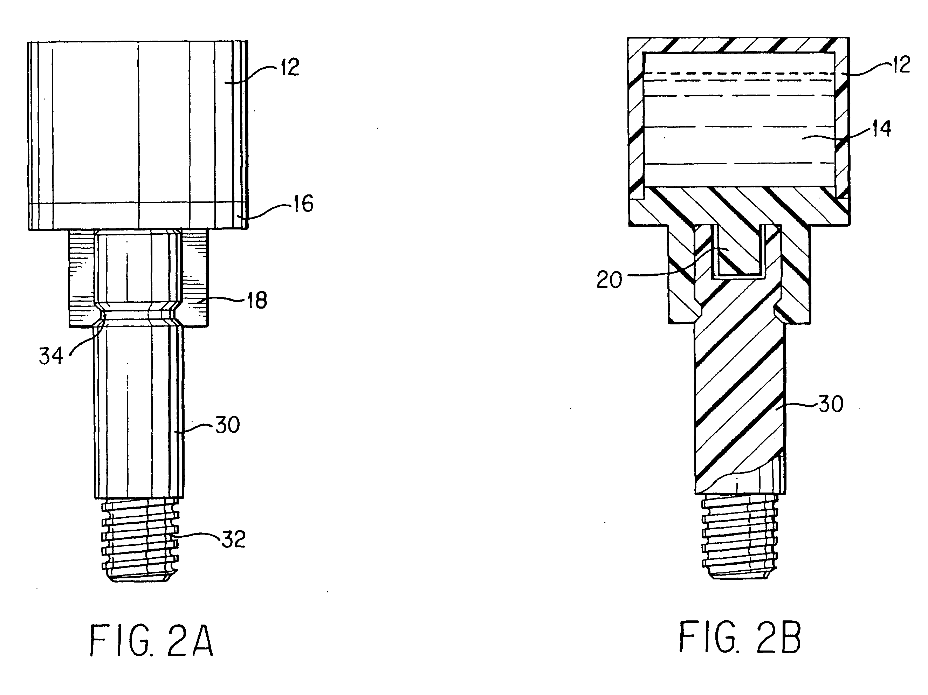

Where a cap is employed, it may be provided with a protruding boss 20 and a plurality (here, three) of snap arms 18, which terminate with inwardly projecting portions 22. The shape and dimensions of the boss are in direct correspondence with the shape and size of the socket 36 provided in the base 30 so as to properly and securely center the imaging marker on the base. The snap arms 18 cooperate with the grooves 34 of the base 30 so as to detachably secure the imaging marker onto the base. The cooperation of these elements is illustrated in FIGS. 2a and 2b. While this example shows the use of snap arms, other fastener structure may be provided for attaching the marker to the base (e.g., screw threads, clasps, hooks, etc.).

The dimensions of the temporary fiducial marker assembly will be somewhat dependent on the state of the art of imaging. The greater the sensitivity of the scanner employed, the lesser the quantity of imaging material necessary to provide a suitable image, which in turn makes it possible to reduce the corresponding size of the marker that must be employed to contain the imaging material. The Applicants have found that a base portion approximately 12mm in length and 2mm-3mm in diameter is sufficiently large to provide for the secure placement of the base into the bone beneath the skin. When the clinician prepares the patient for imaging, the base portion is exposed and an imaging marker approximately 6mm in length is attached to the base; the marker itself may protrude from the scalp and be exposed to air while a scan is performed on the patient. The base and the imaging marker housing are constructed of a bio-compatible organic polymer, such as polyether imide.

FIG. 5 illustrates a second embodiment of the fiducial marker, which may be left implanted entirely beneath the skin for extended periods of time. The marker comprises a cylinder 42 defining a space into which is placed one or more desired imaging agents. As noted in the Summary, a cylindrical shape is preferred, because this shape minimizes the size of the incision that must be made for the marker' s insertion. It is also the shape that best corresponds to the hole that one drills in the bone to accommodate the marker. The body of the cylinder is sealed off with a cap 46 or is otherwise sealed. The body is preferably constructed of an organic polymer known to be well tolerated by the body for extended periods of time, such as polymethyl methacrylate, high density polyethylene, or ceramics such as zirconium oxide and aluminum oxide. The entire marker assembly is small enough for long-term implantation into bone without causing distortion of the bone over time. One exemplary size provides for the marker to be 4 mm in length and 3 mm in diameter.

As shall be explained below, the judicious choice of aqueous imaging agents allows for the construction of a marker that is visible under both CT and MRI imaging modalities. Furthermore, by using a marker that comprises a solid outer portion and an aqueous inner portion, the marker can be located through the use of a non-invasive transcutaneous detection system, such as one employing ultrasound to detect the presence of the solid-liquid interface between the aqueous core and the solid outer portion.

Because the fiducial marker assembly is to be used in a variety of imaging modalities, the use of solid metal is eschewed throughout. The presence of metal may cause unwanted artifacts and image distortion in the image, and may impede efforts to localize the marker (i.e., locate and identify its centroid). The properties characteristic of solid metal, such as high electrical conductivity, paramagnetism, and, for some metals, ferromagnetism, are generally inappropriate for use in MRI.

Although metals have been used in the past as CT markers, they are often generally less than optimal because the high linear attenuation of metals may cause unwanted image artifacts, such as starbursts. While these artifacts can be reduced somewhat by reducing the size of the markers, this reduction in size also reduces the accuracy with which the location of the marker can be determined. In URI, metals cause disturbances in the local magnetic field (the so-called "susceptibility" artifact) which diminish the image intensity and physically shift the position of the image. Such physical phenomena are unsuitable in a fiducial marker. The choice of materials selected as imaging agents is therefore driven by the physics underlying the image modalities that are employed.

In CT studies of human tissue, the brightest anatomical features imaged are bone, which attenuates X-rays more strongly than other tissue. This attenuation is characterized by the so-called "linear attenuation coefficient," which measures the X-ray attenuation per unit of path length. The linear attenuation coefficient increases with increasing electron density (the number of electrons per unit of volume). In order for an X-ray CT imager to produce pixels brighter than bone when imaging a material (such as that of a marker), the material being scanned must have an electron density that is greater than that of bone. Therefore, one approach to enhancing the absorption of X-rays is to increase the electron density per unit volume. This can be accomplished by adding compounds having atoms of high atomic number (Z) to the imaged object, or by substantially increasing the density of the material being scanned. Any high Z value atom suffices. Suitable materials include barium, iodine, titanium, tantalum, silver, gold, platinum, and stainless steel. These materials may be solid, or dissolved as ions in biologically compatible fluids. However, as noted above, the use of solid metal in a fiducial marker tends to create artifacts that degrade the ability of the marker to be used as a true fiducial. These artifacts are higher for markers that have higher linear attenuation coefficients. They are also higher for markers of larger sizes. In particular, for a given linear attenuation coefficient, if the shape and size of the marker is altered so that there is a longer path length through the marker, the tendency to cause an image artifact increases. It is therefore necessary to provide the high Z value material in a diluted form.

One approach to providing an imageable marker of appropriate size that does not yield unwanted artifacts is to dope the marker housing with a CT imaging agent. For example, the housing for the marker, which is normally made of organic polymers, can have barium added as a salt. Alternatively, titanium dioxide may be added to the polymeric housing. Salts of gold or of platinum are also effective materials for rendering the housing shell radio-opaque and thus imageable under CT scanning. Concentrations of these metal salts of up to about 400 mg/ml can be used in the markers without causing appreciable image artifacts. It must be born in mind that it will still be necessary to locate the geometric center of the marker housing so doped; therefore, the geometry of the doped housing should preferably be configured so that its center will be coincident with the center of the volume of any other imaging agent used to accommodate other imaging modalities.

Another approach to providing high Z value agents without resorting to the use of solid metal is to provide them in the form of an aqueous solution. Aqueous solutions of compounds having high Z atoms, such as barium, iodine, titanium, tantalum, silver, platinum, and iron can be used as imaging agents in the fiducial marker of this invention. In particular, compounds of iodine and silver have been found to be effective for this purpose.

For example, in certain preferred embodiments, an aqueous solution of an iodine containing organic molecule with an effective iodine concentration of between about 50 to about 600 mg/ml provides an effective CT imaging agent. In another preferred embodiment, silver nitrate dissolved in water at concentrations of between about 100 mg/ml to about 600 mg/ml is effective. Either solution will be effective in CT at imaging the volume defined by the marker cavity.

By judicious choice of a high-Z aqueous solution, it is possible to create a set of markers having absorption properties in X-ray CT that are anywhere between those of water and 15 times those of bone. However, the effect of these agents on MRI imaging must also be considered, as must the additional requirements that MRI imaging presents.

For example, the use of aqueous solutions where only CT imaging is contemplated is optional - other carriers, such as oils, could be used as carriers for high Z-number elements. However, this is not the case with respect to MRI. The accurate location of MRI markers in biological tissue must be done with an aqueous imaging agent. Substances other than water exhibit different resonance frequencies, or chemical shifts, and will appear in the image displaced from their true location. Therefore, where a common liquid carrier is to be used both for CT as well as MRI imaging agents, it should be water. (The phenomenon of chemical shift is further described in the literature and is well known to practitioners in the art - see e.g., "Edge Artifacts in MR Images: Chemical Shift Effect," Journal of Computer Assisted Tomography 9(2) :252-257 (1985).)

The physics of MRI scanning must further be considered. Pure water is characterized in proton nuclear magnetic resonance by a high spin density and long T1 and T2 values. The relaxation times are in the range of two to four seconds. MRI images are partly based on spin density as well as T1 and T2. The effect of spin density on image brightness is linear; reducing the spin density by one half reduces the brightness by one half. The effects of T1 and T2 are exponential; for example, altering these by one half results in a change of 86% in the brightness in the image. These factors may be summarized mathematically as follows:$\text{I} \text{=} \text{NC} \text{(} {\text{e}}^{\text{-}} {\text{}}^{\text{(}} {\text{}}^{\text{TE}} {\text{}}^{\text{/}} {\text{}}^{\text{T2}} {\text{}}^{\text{)}} \text{) (1-} {\text{e}}^{\text{-(}} {\text{}}^{\text{TR}} {\text{}}^{\text{/}} {\text{}}^{\text{T1}} {\text{}}^{\text{)}} \text{)}$ where I = signal intensity, N = spin density, the parameters TR and TE are the repetition time and the echo time determined by the radio frequency and gradient pulses employed, and C is a constant of proportionality that depends on the scanner and the pulses employed.

These three parameters, T1, T2, and spin density, can be altered by the addition of chemicals selected for their physical properties in solution. One such property is paramagnetism, in which the added material has an unpaired electron in its electron configuration. Such agents shorten the relaxation times T1 and T2 drastically. Another such property is viscosity. Other chemicals may be added to the solution for their ability to alter the viscosity of a solution, even to a point of making a gel. Viscosity is an important consideration, because it is inversely correlated with T2. The more viscous a solution, the greater the number of bonds that are present and the less able the hydrogen nuclei are to react and respond to the magnetic field, which results in a dimmer image. These viscosity enhancing agents dilute the water in the solution, thereby reducing the spin density, and reduce the relaxation times T1 and T2. Whatever the added material, T1 will always be equal to or greater than T2.

Because of the small concentrations of MRI imaging agents appropriate for MRI enhancements, these agents have no significant effect on the CT imaging agent. However, the efficacy of an MRI imaging agent may be obscured by the presence of a CT imaging agent. The MR image is modulated by the cumulative effects of all solutes added to an aqueous medium. This modulation is due to interactions with the hydrogen atoms in water which provide the MR signal. The general effects of three typical CT imaging agents, of one typical MR imaging agent, and of selected combinations of these two types of agents are presented below.

In accordance with the above discussion, it has been determined that silver nitrate, which, as noted above, provides a suitable image under CT scans when provided in concentrations up to about 600 mg/ml, reduces the observed spin density (and hence reduces the brightness of the MR image) by up to 50%. At a preferred concentration of about 600 mg/ml, the observed T1 and T2 values are about 1 second. As the concentration of silver nitrate decreases, the T1 and T2 values approach those of pure water. This observed effect affords the opportunity to provide a variety of marker compositions based on silver nitrate (as the CT agent) to create differing contrast levels with respect to human tissue under MRI.

A solution of iohexol (a non-ionic X-ray CT imaging agent) in concentrations of up to about 600 mg/ml of iodine also reduces the observed spin density by up to 50%. At a concentration of 150 mg of iodine per ml of aqueous solution, the observed T1 value is 300-400 milliseconds, and the observed T2 value is 100-120 milliseconds. The effect on these parameters is in accord with similar effects noted from increasing the viscosity of aqueous solutions. Over the entire range of iohexol concentrations, it has been determined that one can alter the spin density by 50%, change the T1 value from 4 seconds to 0.15 seconds, and the T2 value from 4 seconds to 0.04 seconds. Thus, iohexol too, may be used to provide a variety of marker compositions to create different contrast levels with respect to human tissue under MRI.

A solution of iothalamate meglumine (an ionic X-ray CT imaging agent) in concentrations up to about 600 mg/ml of iodine reduces the observed spin density by factors ranging up to 75%. At a concentration of 175 mg of iodine per ml of aqueous solution, the observed T1 value is 1200-1500 milliseconds, and the observed T2 value is 300-350 milliseconds. The solution is not viscous at this concentration. Over the entire range of iothalamate meglumine concentrations it has been determined that one can alter proton spin by 75%, change T1 value from 4 seconds to 1 second, and the T2 value from 4 seconds to 200 milliseconds. Thus iothalamate meglumine may be used to provide a variety of marker compositions to create different contrast with respect to human tissue.

Within limits, one can prepare solutions of reduced spin density in which T1 is equal to T2, and solutions providing spin density in which T1 is greater that T2. T1 and T2 values of these altered solutions range from 1 millisecond to 4 seconds, while the spin density ranges from 0 molar (no water at all) to 111 molar in hydrogen (pure water). The instant invention identifies a number of substances suitable for use in MRI imaging in fiducial markers. One preferred suitable MRI imaging agent is gadopentetate dimeglumine. Another possible MRI imaging agent is gadoteridol. (Each of the aforementioned substances has received FDA approval for use as an injectable MRI imaging agent.) Other possible agents for use as MRI imaging agents include Ferric Chloride (FeCl₃) and Copper Sulfate (CuSO₄) in concentrations of between 0.5 mM and 5 mM. For a more complete listing of contrast agents and their properties, see chapter 14 of Magnetic Resonance Imaging. 2nd ed., edited by Stark and Bradley, 1992, the contents of which are herein incorporated by reference.

A solution of gadopentetate dimeglumine-DPTA (an injectable MR contrast agent) in concentrations up to about 0.5 mM (millimolar) is seen to have little effect on the observed spin density of the solution. At a concentration of 0.5 mM, the observed T1 value is 50 to 100 milliseconds, and the observed T2 value is 8 to 15 milliseconds. Over the entire range of concentrations one observes spin density of 111 molar in hydrogen (water). T1 varies from 50 milliseconds to 4 seconds, and T2 varies from 8 milliseconds to 4 seconds. This is in accord with the reported effect of the paramagnetic material. By varying the marker compositions based on the gadopentetate compound, it is possible to create a variety of different contrasts with respect to human tissue.

The four solutions just discussed were based on one solute in water. To provide a multi-modality marker, binary mixtures of CT and MR contrast agents are considered. It has been observed that binary combinations of gadopentetate-DPTA with any of the other chemical compounds set forth produce a synergistic effect. The matrix of possibilities for altering spin density, T1 and T2, by varying the concentration of MRI and CT imaging agents, enhances the ability to tailor solutions to give maximum contrast in all three types of MR image parameters. For example, with respect to the permanent marker, iothalamate meglumine at a concentration of 175 mg/ml combined with gadopentetate meglumine-DPTA at 0.5 mM creates a solution with the following MR properties: a spin density corresponding to 75-80 molar water, T1 value of 400-500 milliseconds, and a T2 value of 150-200 milliseconds. A variety of other binary compositions permit one to create many different ratios of spin density, T1 and T2 values.

Hence, as these examples suggest, it is possible to create a set of MR markers with any combination of spin density, T1 and T2 that are smaller in magnitude than those of water. The optimum values of these parameters as specified by the marker application dictate the composition of the solution.

For one marker to be optimized for both MRI and CT, the imaging agents and their concentrations must be selected such that the solution and/or housing may be differentiated in the X-ray by its radio-opacity and at the same time be differentiated in the MRI by its MRI parameter set - spin density, T1, and T2. As noted above, it is known that aqueous solutions of compounds based on high Z number elements will provide the necessary degree of radio-opacity for CT imaging. It is also known that such substances may reduce the imaging efficacy of compounds selected for their use as MRI contrast agent, such as gadopentetate dimeglumine-DPTA. In order to combine the two agents in an effective manner, they are mixed together in varying concentration and tested under both CT and MRI scans until one has empirically determined a concentration of each that provides a marker that is acceptably imageable under both modalities.

As a result of such a course of testing, the Applicants have identified two preferred binary mixtures that meet these requirements when used in the permanent marker; these are iothalamate meglumine (175 mg of iodine/ml) with gadopentetate dimeglumine-DPTA (0.5 mM) and silver nitrate (350 mg/ml) with gadopentetate dimeglumine-DPTA (0.5 mM). In the case of the temporary marker, the concentrations of the CT imaging agents are reduced to 165 mg of iodine per ml of aqueous solution of iothalamate meglumine and 200 mg/ml of silver nitrate respectively.

In an alternative the aqueous solutions used as the carrier for the imaging agents could have the characteristics of a gel. The imaging agents could be provided as mixtures of three or more compounds selected to optimize particular imaging characteristics.

Further a flatter, more disk-like marker than that which is shown in the figures may be employed. Increasing the largest dimension of X-ray traversal boosts the brightness of the image in CT. It also allows one to take CT scans with thicker slices in which the image of the marker does not become lost in the corresponding pixel. This is especially useful in trauma cases, when there is not sufficient time for more refined views.

In another variant, the imaging agents discussed above can be relied upon for one imaging mode and another technique relied on for locating the marker in a second imaging mode. For example, one may image a marker under a first modality (e.g., MRI) using the imaging agents discussed above, and then locate the marker in the image space generated by a second imaging modality (e.g., CT) by physically locating the marker in the physical space of the second imaging machine. For example, robotic arms such as are disclosed in U.S. Patent No. 5,142,930 (the contents of which are herein incorporated by reference) or U.S. Patent No. 4,991,579 could be used for this purpose, since in the course of a scan the addresses of each point in image space are generally defined with respect to the imaging machine and hence to any arm (such as that disclosed in the ' 930 patent) or other device whose location is clearly defined with respect to that machine. One may then label the corresponding point in image space as containing the location of marker, even where it is not directly imageable in that imaging mode.

## Claims

1. A fiducial marker assembly, the assembly comprising:
an imaging marker (10) having a non metallic housing (12) of a biocompatible material,
the housing (12) including a cavity (14) containing a mixture of agents,
wherein the mixture comprises two agents which constitute respective imaging materials for mutually different imaging modalities, and in that the respective centroids of said respective imaging materials are substantially coincident.

2. A fiducial marker assembly, the assembly comprising:
an imaging marker (10) having a non metallic housing (12) of a biocompatible material, and doped with a first agent,
the housing (12) further including a cavity (14) containing a second agent,
wherein both agents each constitute respective imaging materials for mutually different imaging modalities, and in that the respective centroids of said respective imaging materials are substantially coincident.

3. The device of claims 1 or 2, **characterised in that** it further comprises:
a base (30) for supporting the imaging marker (10), said base (30) having means for accommodating the attachment of the base (30) to tissue;
a connector for detachably securing the imaging marker (10) with the base (30); and
means for sealing the cavity (14) of the imaging marker (10) against leakage of imaging material contained within the cavity (14).

4. The device of claim 3 **characterised in that** the means for sealing (24) the cavity (14) is a cap (16) that is securely connected to the housing of the imaging marker (10).

5. The device of claim 4, **characterised in that** the cap (16) includes a protruding boss (20) and the base includes a corresponding socket (36,38) sized so that the boss (20) of the cap (16) can mate with the socket (36,38) and thereby form a link between the cap (16) and the base (30).

6. The device of claims 3, 4 or 5 **characterised in that** the sealing means (24) is provided with a plurality of extending arms (18) for detachable attachment with the base (30).

7. The device of claims 3, 4 or 5 **characterised in that** the base (30) includes threads (32) for facilitating its attachment to tissue.

8. The device of claim 6 **characterised in that** the base (30) is provided with grooves (34) and the extending arms (18) of the sealing means (24) terminate in projecting portions (22) that cooperate with the grooves (34) on the base for connection therewith.

9. The device of any one of the preceding claims **characterised in that** the marker housing is cylindrical in shape.

10. The device of claim 9 **characterised in that** the marker housing is formed of a polymer.

11. The device of claim 2 **characterised in that** the marker housing is doped with a first agent in an amount effective to render the marker imageable in a computed tomographic scan.

12. The device of claim 11 **characterised in that** the first agent is a radio-opaque substance having a high atomic number.

13. The device of claim 12 **characterised in that** the compound is selected from the group of elements consisting of barium, titanium, silver, gold, and platinum.

14. The device of claim 13 **characterised in that** the element is provided as a salt.

15. The device of claim 14 **characterised in that** the salt is provided in a concentration of up to about 400 mg/ml.

16. The device of claim 1 **characterised in that** the cavity contains a first agent comprising a compound having a high atomic number.

17. The device of claim 16 **characterised in that** the compound is selected from the group of elements consisting of barium, iodine, titanium, gold, tantalum, silver, platinum, and iron.

18. The device of claims 16 or 17 **characterised in that** the concentration of the element provided in the first agent is present in an amount effective to render the volume filled by the agent radio-opaque.

19. The device of claim 18 **characterised in that** the first agent contains ( a) iohexol or (b) silver nitrate or (c) iothalamate meglumine.

20. The device of claim 18 **characterised in that** the concentration of silver nitrate is about 350 mg/ml.

21. The device of claim 18 **characterised in that** the concentration of silver nitrate is about 200 mg/ml, and wherein the cavity contains a second agent comprising gadopentetate dimeglumine-DPTA at a concentration of about 0.5 mM.

22. The device of claim 18 **characterised in that** the iothalamate is provided as iothalamate meglumine.

23. The device of claim 22 **characterised in that** the concentration of iothalamate meglumine is about 175 mg of iodine/ml, and wherein the cavity contains a second agent comprising gadopentetate dimeglumine-DPTA at a concentration of about 0.5 mM.

24. The device of claim 18 **characterised in that** the concentration of iothalamate meglumine is about 165 mg of iodine/ml.

25. The device of claims 1-5 **characterised in that** said second agent, is imageable under nuclear magnetic resonance imaging (MRI).

26. The device of claim 25 **characterised in that** the agent comprises gadopentetate dimeglumine-DPTA.

27. The device of claim 26 **characterised in that** the gadopentetate dimeglumine-DPTA is provided at a concentration of about 0.5mM.

28. The device of claim 1 **characterised in that** the first and second agents are aqueous and miscible to form a mixture within the cavity (14) wherein the first agent is radio-opaque and the second agent is imageable under nuclear magnetic resonance.

29. The device of claim 28 **characterised in that** the first agent is silver nitrate and the second agent is gadopentetate dimeglumine-DPTA.

30. The device of claim 29 **characterised in that** the concentration of the silver nitrate is about 350 mg/ml, and the concentration of the gadopentetate dimeglumine-DPTA is about 0.5 mM.

31. The device of claim 28 **characterised in that** the first agent is iothalamate meglumine and the second agent is gadopentetate dimeglumine-DPTA.

32. The device of claim 31 **characterised in that** the concentration of the iothalamate meglumine is about 175 mg of iodine/ml, and the concentration of the gadopentetate dimeglumine-DPTA is about 0.5 mM.

33. The device of claims 1 or 2 **characterised in that** the fiducial marker assembly is sized so as to permit the long-term implantation of the assembly into a patient without causing the distortion of the tissue during the period during which the device is implanted.

34. A device according to any one of the preceding claims **characterised in that** the fiducial marker has a base that in use is secured to tissue and at least one imaging marker (10) is provided that can be detachably secured to said base

35. The device of claim 34 **characterised in that** a plurality of said markers is provided, each of which has been optimised for imaging under at least one imaging modality.

36. The device of claim 1, **characterised in that** the imaging marker (10) contains a radioactive isotope.

37. A method for providing a fiducial marker having a non-metallic housing of a biocompatible material that is imageable under different imaging modalities, including computerized X-ray tomography and nuclear magnetic resonance imaging, wherein the marker has a single cavity for receiving imaging material comprising the steps of:
providing the marker with a first imaging agent that is imageable under computerized X-ray tomography; and providing the cavity of the marker with a second imaging agent that is imageable under nuclear magnetic resonance imaging,
wherein the centres of the regions of the fiducial marker that are defined by each imaging agent are coincident, thereby permitting the proper registration of images obtained by each imaging modality.

## Patentansprüche

1. Vorrichtung zur Markierung als Bezugspunkt, bestehend aus:
einem Bildanzeiger (10) mit einem nichtmetallischen Gehäuse (12) aus biokompatiblem Material, wobei das Gehäuse (12) einen Hohlraum (14) aufweist, der eine Mischung eines Mittels enthält, und die Mischung zwei Medien aufweist, welche jeweils Bildmaterialien für gegenseitig unterschiedliche Bildmodalitäten darstellen und wobei die diesbezüglichen Flächenschwerpunkte der diesbezüglichen Bildmaterialien im wesentlichen übereinstimmen.

2. Vorrichtung zur Markierung als Bezugspunkt, bestehend aus:
einem Bildanzeiger (10) mit einem nichtmetallischen Gehäuse (12) aus biokompatiblem Material, und dotiert mit einem ersten Medium, wobei das Gehäuse (12) einen Hohlraum (14) aufweist, der ein zweites Mittel aufweist, wobei beide Mittel jeweils Bildmaterialien für gegenseitig unterschiedliche Bildmodalitäten darstellen und wobei die diesbezüglichen Flächenschwerpunkte der entsprechenden Bildmaterialien im wesentlichen übereinstimmen.

3. Vorrichtung nach den Ansprüchen 1 oder 2, **dadurch gekennzeichnet, dass** sie zur Stützung des Bildanzeigers (10) einen Sockel (30) aufweist, welcher mit Mitteln zur Anpassung der Befestigung des Sockels (30) an ein Gewebe versehen ist;
einen Verbinder zur abnehmbaren Sicherung des Bildanzeigers (10) von dem Sockel (30); und
Mittel zur Abdichtung des Hohlraums (14) des Bildanzeigers (10) gegen Leckage des in dem Hohlraum (14) enthaltenen Bildmaterials.

4. Vorrichtung nach Anspruch 3, **dadurch gekennzeichnet, dass** das Mittel zur Abdichtung (24) des Hohlraumes (14) ein Deckel (16) ist, der mit dem Gehäuse des Bildanzeigers (10) sicher verbunden ist.

5. Vorrichtung nach Anspruch 4, **dadurch gekennzeichnet, dass** der Deckel (16) einen herausragenden Vorsprung (20) aufweist und der Sockel (30) schließt eine entsprechende Fassung (36,38) ein, welche so dimensioniert ist, dass der Vorsprung (20) des Deckels (16) mit der Fassung (36, 38) zusammenpaßt, wodurch eine Verkettung zwischen dem Deckel (16) und dem Sockel (30) erfolgt.

6. Vorrichtung nach den Ansprüchen 3, 4 oder 5, **dadurch gekennzeichnet, dass** die Dichtungsmittel (24) mit mehreren lang gestreckten Armen (18) zur lösbaren Befestigung mit dem Sockel (20) versehen sind.

7. Vorrichtung nach Anspruch 3, 4 oder 5, **dadurch gekennzeichnet, dass** der Sockel (30) ein Gewinde (32) zur leichten Befestigung mit dem Gewebe aufweist.

8. Vorrichtung nach Anspruch 6, **dadurch gekennzeichnet, dass** der Sockel (30) mit Schlitzen (34) versehen ist, wobei die langgestreckten Arme (18) der Dichtungsmittel (24) in Form von vorspringenden Teilen (22) enden, die mit den Schlitzen im Sockel zur Befestigung zusammenwirken.

9. Vorrichtung nach einem der voranstehenden Ansprüche, **dadurch gekennzeichnet, dass** das Gehäuse des Anzeigers eine zylindrische Form aufweist.

10. Vorrichtung nach Anspruch 9, **dadurch gekennzeichnet, dass** das Gehäuse des Anzeigers aus einem Polymer besteht.

11. Vorrichtung nach Anspruch 2, **dadurch gekennzeichnet, dass** das Gehäuse des Anzeigers mit einem ersten Mittel in einer Menge gestrichen ist, die wirksam ist, um ihn in einer computertomographischen Abtastung sichtbar zu machen.

12. Vorrichtung nach Anspruch 11, **dadurch gekennzeichnet, dass** das erste Mittel eine röntgenstrahlenundurchlässige Substanz aus Stoffen ist, welche ein hohes Atomgewicht aufweisen.

13. Vorrichtung nach Anspruch 12, **dadurch gekennzeichnet, dass** die Stoffe aus der Gruppe der Elemente ausgewählt sind, welche Barium, Titan, Silber, Gold und Platin enthält.

14. Vorrichtung nach Anspruch 13, **dadurch gekennzeichnet, dass** das Element als Salz aufbereitet ist.

15. Vorrichtung nach Anspruch 14, **dadurch gekennzeichnet, dass** das Salz in einer Konzentration bis zu etwa 400 mg/ml vorliegt.

16. Vorrichtung nach Anspruch 1, **dadurch gekennzeichnet, dass** der Hohlraum einen ersten Stoff einer Mischung mit hohem Atomgewicht enthält.

17. Vorrichtung nach Anspruch 16, **dadurch gekennzeichnet, dass** die Mischung aus einer Gruppe von Elementen ausgewählt ist, die aus Barium, Jod, Titan, Gold, Tantal, Silber, Platin und Eisen besteht.

18. Vorrichtung nach Anspruch 16 oder 17, **dadurch gekennzeichnet, dass** die Konzentration des Elements, welches in dem ersten Stoff vorgesehen ist, in einer Menge vorhanden ist, welche das durch den Stoff aufgefüllte Volumen für Röntgenstrahlen undurchlässig macht (Röntgenkontrastmittel).

19. Vorrichtung nach Anspruch 18, **dadurch gekennzeichnet, dass** der erste Stoff (a) Iohexol oder (b) Silbernitrat oder (c) lothalamat oder Meglumine ist.

20. Vorrichtung nach Anspruch 18, **dadurch gekennzeichnet, dass** die Konzentration des Silbernitrats etwa 350 mg/ml beträgt.

21. Vorrichtung nach Anspruch 18, **dadurch gekennzeichnet, dass** die Konzentration des Silbernitrats etwa 200 mg/ ml beträgt, und wobei der Hohlraum einen zweiten Stoff enthält, welcher Gadopentetat Dimiglumin-DPTA in einer Konzentration von 0,5 mM aufweist.

22. Vorrichtung nach Anspruch 18, **dadurch gekennzeichnet, dass** das lothalamat als lothalamat Meglumin vorgesehen ist.

23. Vorrichtung nach Anspruch 22, **dadurch gekennzeichnet, dass** die Konzentration des Iothalamats Meglumin beträgt etwa 175 mg an lodin/ml und wobei der Hohlraum einen zweiten Stoff enthält, der Gadopentetat Dimiglumin -DPDA in einer Konzentration von etwa 0,5 mM vorliegt.

24. Vorrichtung nach Anspruch 18, **dadurch gekennzeichnet, dass** die Konzentration des Jothalamat Meglumin etwa 165 mg von Iodine/ml beträgt.

25. Vorrichtung nach den Ansprüchen 1-5, **dadurch gekennzeichnet, dass** der zweite Stoff bei Magnetkernresonanz sichtbar ist.

26. Vorrichtung nach Anspruch 25, **dadurch gekennzeichnet, dass** der Stoff Gadopentetat Dmieglumin-DPTA enthält.

27. Vorrichtung nach Anspruch 26, **dadurch gekennzeichnet, dass** das Gadopentetat-Dimiglumin-DPTA in einer Konzentration von 0,5 mM vorgesehen ist.

28. Vorrichtung nach Anspruch 1, **dadurch gekennzeichnet, dass** der erste und zweite Stoff flüssig und mischbar ist, um innerhalb des Hohlraums (14) eine Mischung zu bilden,
wobei der erste Stoff röntgenstrahlenundurchlässig und der zweite Stoff unter Magnetkernresonanz sichtbar ist.

29. Vorrichtung nach Anspruch 28, **dadurch gekennzeichnet, dass** der erste Stoff aus Silbernitrat und der zweite aus Gadopentetat-Dimeglumin-DPTA besteht.

30. Vorrichtung nach Anspruch 29, **dadurch gekennzeichnet, dass** die Konzentration des Silbernitrats etwa 350 mg/ml beträgt und die Konzentration des Gadopentetate Dimeglumin-DPTA etwa 0,5 mM beträgt.

31. Vorrichtung nach Anspruch 28, **dadurch gekennzeichnet, dass** der erste Stoff aus Isothalamat Meglumin und der zweite Stoff aus Gadopentetat Dimeglumin-DPTA besteht

32. Vorrichtung nach Anspruch 31, **dadurch gekennzeichnet, dass** die Konzentration des Isothalamat Meglumin etwa 175 mg bezogen auflodin/ ml beträgt und die Konzentration des Gadopentetat Dimeglumin-DPTA etwa 0,5 mM beträgt,

33. Vorrichtung nach den Ansprüchen 1 oder 2, **dadurch gekennzeichnet, dass** der Anzeiger so groß ausgebildet ist, dass eine Langzeitimplantation der Anzeigevorrichtung in einen Patienten durchführbar ist, ohne eine Störung des Gewebes während des Zeitraumes während dem die Anzeigevorrichtung implantiert ist befürchten zu müssen.

34. Vorrichtung nach einem der voranstehenden Ansprüche, **dadurch gekennzeichnet, dass** der Anzeiger einen Sockel aufweist, der im Benutzungszustand mit dem Gewebe gesichert ist, wobei mindestens ein Bildanzeiger (10) vorgesehen ist, der mit dem Sockel lösbar befestigt ist.

35. Vorrichtung nach Anspruch 34, **dadurch gekennzeichnet, dass** eine Vielzahl Anzeiger vorgesehen ist, wobei jeder Anzeiger optimiert ist, um bei einer Markierungsmodalität eine Markierung aufzuweisen.

36. Vorrichtung nach Anspruch 1, **dadurch gekennzeichnet, dass** der Bildanzeiger (10) ein Radioaktives Isotop enthält.

37. Verfahren zum Einsatz eines Vergleichsanzeigers aus einem nichtmetallischen Gehäuse und biokompatiblem Material, welcher unter verschiedenen Anregungen Markierungen aussendet, nämlich Anregungen aus einer Komputer-Röntgenstrahlen-Tomographie, einschließlich Kernmagnetresonanzen, wobei der Anzeiger einen einzigen Hohlraum, zur Aufnahme von Bildmatcrial aufweist, mit folgenden Schritten:
Zugabe eines ersten Stoffes an den Anzeiger, der auf Röntgenstrahlen-Komputertomographie anspricht, und Zugabe eines zweiten Stoffes in den Hohlraum des Anzeigers, der auf Kernmagnetresonanzen anspricht,
wobei die Zentren der Berciche des Anzeigers, die durch jeden Anregungsstoff definiert sind koinzident sind, wodurch die genaue Registricrung der durch jede Anregungsmodalität erhaltene Anregung ermöglicht wird.

## Revendications

1. Assemblage de repère de calage, l'assemblage comprenant :
un repère d'imagerie (10) ayant un logement non métallique (12) d'un matériau biocompatible,
le logement (12) comprenant une cavité (14) contenant un mélange d'agents, dans lequel le mélange comprend deux agents qui constituent des matériaux d'imagerie respectifs pour des modalités d'imagerie mutuellement différentes, et les centroïdes desdits matériaux d'imagerie respectifs sont substantiellement coïncidents.

2. Assemblage de repère de calage, l'assemblage comprenant :
un repère d'imagerie (10) ayant un logement non métallique (12) d'un matériau biocompatible, et additionné d'un premier agent,
le logement (12) comprenant en outre une cavité (14) contenant un second agent, dans lequel les deux agents constituent chacun des matériaux d'imagerie respectifs pour des modalités d'imagerie mutuellement différentes, et les centroïdes respectifs desdits matériaux d'imagerie respectifs sont substantiellement coïncidents.

3. Dispositif selon la revendication 1 ou 2, **caractérisé en ce qu'**il comprend en outre :
une base (30) destinée à supporter le repère d'imagerie (10), ladite base (30) ayant des moyens d'accommodation de la fixation de la base (30) au tissu ;
un raccord pour fixer de manière détachable le repère d'imagerie (10) à la base (30) ; et
des moyens de scellement de la cavité (14) du repère d'imagerie (10) pour empêcher les fuites du matériau d'imagerie contenu dans la cavité (14).

4. Dispositif selon la revendication 3, **caractérisé en ce que** les moyens de scellement (24) de la cavité (14) sont un capuchon (16) qui est connecté de manière sûre au logement du repère d'imagerie (10).

5. Dispositif selon la revendication 4, **caractérisé en ce que** le capuchon (16) comprend un bosselage protubérant (20) et la base comprend une douille correspondante (36, 38) dimensionnée de manière à ce que le bosselage (20) du capuchon (16) puisse s'apparier avec la douille (36, 38) et ainsi former un lien entre le capuchon (16) et la base (30).

6. Dispositif selon les revendications 3, 4 ou 5, **caractérisé en ce que** les moyens de scellement (24) sont munis d'une pluralité de bras extensibles (18) pour une fixation détachable avec la base (30).

7. Dispositif selon les revendications 3, 4 ou 5, **caractérisé en ce que** la base (30) comprend des fils (32) destinés à faciliter sa fixation au tissu.

8. Dispositif selon la revendication 6, **caractérisé en ce que** la base (30) est munie de sillons (34) et les bras extensibles (18) des moyens de scellement (24) se terminent en des parties de projection (22) qui coopèrent avec les sillons (34) sur la base pour le raccord à celle-ci.

9. Dispositif selon l'une quelconque des revendications précédentes, **caractérisé en ce que** le logement du repère est de forme cylindrique.

10. Dispositif selon la revendication 9, **caractérisé en ce que** le logement du repère est formé d'un polymère.

11. Dispositif selon la revendication 2, **caractérisé en ce que** le logement du repère est additionné d'un premier agent en une quantité efficace pour rendre imageable le repère sur un tomodensitogramme.

12. Dispositif selon la revendication 11, **caractérisé en ce que** le premier agent est une substance radio-opaque ayant un nombre atomique élevé.

13. Dispositif selon la revendication 12, **caractérisé en ce que** le composé est choisi dans le groupe d'éléments comprenant le baryum, le titane, l'argent, l'or, et le platine.

14. Dispositif selon la revendication 13, **caractérisé en ce que** l'élément est fourni sous la forme d'un sel.

15. Dispositif selon la revendication 14, **caractérisé en ce que** le sel est fourni dans une concentration allant jusqu'à environ 400 mg/ml.

16. Dispositif selon la revendication 1, **caractérisé en ce que** la cavité contient un premier agent comprenant un composé ayant un nombre atomique élevé.

17. Dispositif selon la revendication 16, **caractérisé en ce que** le composé est choisi dans le groupe d'éléments comprenant le baryum, l'iode, le titane, l'or, le tantale, l'argent, le platiné et le fer.

18. Dispositif selon la revendication 16 ou 17, **caractérisé en ce que** la concentration de l'élément fourni dans le premier agent est présente dans une quantité efficace pour rendre radio-opaque le volume rempli par l'agent.

19. Dispositif selon la revendication 18, **caractérisé en ce que** le premier agent contient (a) de l'iohexol ou (b) du nitrate d'argent ou (c) de l'iothalamate de méglumine.

20. Dispositif selon la revendication 18, **caractérisé en ce que** la concentration du nitrate d'argent est d'environ 350 mg/ml.

21. Dispositif selon la revendication 18, **caractérisé en ce que** la concentration du nitrate d'argent est d'environ 200 mg/ml, et dans lequel la cavité contient un second agent comprenant du gadopentétate de diméglumine-DPTA à une concentration d'environ 0,5 mM.

22. Dispositif selon la revendication 18, **caractérisé en ce que** l'iothalamate est fourni sous la forme d'iothalamate de méglumine.

23. Dispositif selon la revendication 22, **caractérisé en ce que** la concentration d'iothalamate de méglumine est d'environ 175 mg d'iode/ml, et dans lequel la cavité contient un second agent comprenant du gadopentétate de diméglumine-DPTA à une concentration d'environ 0,5 mM.

24. Dispositif selon la revendication 18, **caractérisé en ce que** la concentration d'iothalamate de méglumine est d'environ 165 mg d'iode/ml.

25. Dispositif selon les revendications 1 à 5, **caractérisé en ce que** ledit second agent est imageable par imagerie par résonance magnétique nucléaire (IRM).

26. Dispositif selon la revendication 25, **caractérisé en ce que** l'agent comprend du gadopentétate de diméglumine-DPTA.

27. Dispositif selon la revendication 26, **caractérisé en ce que** le gadopentétate de diméglumine-DPTA est fourni à une concentration d'environ 0,5 mM.

28. Dispositif selon la revendication 1, **caractérisé en ce que** le premier et le second agents sont aqueux et miscibles pour former un mélange à l'intérieur de la cavité (14), dans lequel le premier agent est radio-opaque et le second agent est imageable par résonance magnétique nucléaire.

29. Dispositif selon la revendication 28, **caractérisé en ce que** le premier agent est du nitrate d'argent et le second agent est du gadopentétate de diméglumine-DPTA.

30. Dispositif selon la revendication 29, **caractérisé en ce que** la concentration du nitrate d'argent est d'environ 350 mg/ml, et la concentration du gadopentétate de diméglumine-DPTA est d'environ 0,5 mM.

31. Dispositif selon la revendication 28, **caractérisé en ce que** le premier agent est de l'iothalamate de méglumine et le second agent est du gadopentétate de diméglumine-DPTA.

32. Dispositif selon la revendication 31, **caractérisé en ce que** la concentration de l'iothalamate de méglumine est environ 175 mg d'iode/ml, et la concentration du gadopentétate de diméglumine-DPTA est environ 0,5 mM.

33. Dispositif selon la revendication 1 ou 2, **caractérisé en ce que** l'assemblage de repère de calage est dimensionné de manière à permettre l'implantation à long-terme de l'assemblage dans un patient sans entraîner de déformation du tissu au cours de la période pendant laquelle le dispositif est implanté.

34. Dispositif selon l'une quelconque des revendications précédentes, **caractérisé en ce que** le repère de calage a une base qui durant l'utilisation est fixée au tissu et au moins un repère d'imagerie (10) est fourni qui peut être fixé de manière détachable à ladite base.

35. Dispositif selon la revendication 34, **caractérisé en ce qu'**une pluralité desdits repères est fournie, chacun d'entre eux a été optimisé pour l'imagerie dans au moins une modalité d'imagerie.

36. Dispositif selon la revendication 1, **caractérisé en ce que** le repère d'imagerie (10) contient un isotope radioactif.

37. Procédé de fourniture d'un repère de calage ayant un logement non métallique d'un matériau biocompatible qui est imageable dans différentes modalités d'imagerie, y compris la tomodensitométrie par rayons X et l'imagerie par résonance magnétique nucléaire, dans lequel le repère a une cavité simple destinée à recevoir le matériau d'imagerie comprenant les étapes consistant à :
fournir un repère avec un premier agent d'imagerie qui est imageable par tomodensitométrie par rayons X ; et munir la cavité du repère d'un second agent d'imagerie qui soit imageable par imagerie par résonance magnétique nucléaire,
dans lequel les centres des régions du repère de calage qui sont définis par chaque agent d'imagerie sont coïncidents, permettant ainsi l'enregistrement correct des images obtenues par chaque modalité d'imagerie.
